# EUROPÄISCHE PATENTSCHRIFT

(11) **EP 1 430 845 B1**
(45) Veröffentlichungstag und Bekanntmachung des Hinweises auf die Patenterteilung: **08.07.2009**
(21) Anmeldenummer: 03024303.4
(22) Anmeldetag: 24.10.2003
(51) Int. Cl.: A61B 17/74

(54) **Osteosynthesehilfsmittel mit Marknagel, Verriegelungsschraube und Vorspannhülse**
Means for osteosynthesis comprising medullary nail, screw and spring sleeve
Moyen d' osteosynthese comprenant un clou intramedullaire, une vis et un manchon élastique

(30) Priorität: 19.12.2002 DE 20219683 U
(43) Veröffentlichungstag der Anmeldung: 23.06.2004
(73) Patentinhaber: Stryker Trauma GmbH, 24232 Schönkirchen (DE)
(72) Erfinder: Zander, Nils, 24340 Eckernförde (DE); Cremer, Axel, 23795 Fahrenkrog (DE); Seemann, Michael, 24145 Kiel (DE)
(74) Vertreter: Kopf, Korbinian Paul

(56) Entgegenhaltungen:
- DE-A- 19 852 945
- US-A- 4 959 064
- US-A1- 2002 156 473
- US-A1- 2002 198 527

## Beschreibung

Die Erfindung bezieht sich auf ein Osteosynthesehilfsmittel für Röhrenknochen nach dem Anspruch 1.

Insbesondere bezieht sich die Erfindung auf ein Verriegelungsnagelsystem, wie es für Röhrenknochen, insbesondere die Femur, die Tibia oder den Humerus bekannt geworden ist. Das Grundprinzip besteht aus einem länglichen Nagelschaft, der an den Enden jeweils mindestens eine Querbohrung aufweist. Durch die Querbohrung hindurch wird eine Knochen- bzw. eine Verriegelungsschraube geführt, mit der der Nagelschaft im Knochen sowohl in Axial- als auch in Drehrichtung festgelegt wird.

Ein besonderes Problem bei der Anwendung derartiger Verriegelungsnägel, beispielsweise bei Humeruskopf-Frakturen, stellt das postoperative Auswandern der Schrauben dar. Durch Verrutschen der Fragmente oder Resorption des Knochens kann die Vorspannung, die beim Einschrauben der Verriegelungsschraube erzeugt wird, verloren gehen und dazu führen, dass sich die Verriegelungsschraube herausdreht.

Bei der Anwendung des Verriegelungsnagels als Suprakondylarnagel ist bekannt, durch die Kondylen eine so genannte Kondylenschraube hindurchzuführen. Sie besteht üblicherweise aus einer Kondylenschraube, die einen relativ langen Schaft aufweist und einen Gewindeabschnitt sowie einer Kondylenmutter, die auf den Schaft aufgeschraubt ist. Die Kondylenmutter kann einen hülsenförmigen Abschnitt aufweisen, sodass zwischen der Mutter und dem Kopf der Schraube ein glatter Schaft gebildet sein kann. Bei einer derartigen Versorgung kann geschehen, dass ebenfalls aufgrund von Resorption von Knochenteilen oder Verlagerung von Fragmenten eine Sicherung des Nagelschaftes nicht mehr in ausreichendem Maße gewährleistet ist, er zum Beispiel zur Seite hin wandert.

Der Erfindung liegt die Aufgabe zugrunde, ein Osteosynthesehilfsmittel zu schaffen, bei dem sichergestellt ist, dass die ursprünglich aufgebrachte Spannung auf eine Verriegelungsschraube oder eine Kondylenschraube auch nach einiger Zeit im Wesentlichen aufrechterhalten bleibt.

Diese Aufgabe wird durch die Merkmale des Anspruchs 1 gelöst, wobei die im einleitenden Teil definierten Merkmale z.B. aus dem Dokument DE-A-198 52 945 bekannt sind.

Bei der Erfindung ist zwischen dem Kopf der Verriegelungsschraube und dem Nagelschaft eine Vorspannhülse angeordnet, die in Achsrichtung federnd nachgebend ist. Nach einer Ausgestaltung der Erfindung kann die Hülse an einem Ende einen radialen Flansch aufweisen, gegen den der Kopf der Verriegelungsschraube zur Anlage kommt.

Bei der Anwendung der Erfindung ist das Loch, das nach dem Auffinden der Querbohrung im Nagelschaft im Knochen gebohrt werden muss, mit einem Durchmesser zu versehen, dass auch die Hülse eingeführt werden kann. Die Hülse ist so bemessen, dass sie sich gegen den Nagelschaft anlegt und dabei mit ihrem Flansch an der äußeren Kortikalis anliegt. Wird nun die Verriegelungsschraube eingedreht und gespannt, wird die Vorspannhülse unter axiale Vorspannung gesetzt. Diese Vorspannung sorgt dafür, dass sie auch dann erhalten bleibt, wenn aufgrund von Veränderungen im Knochen der Abstand zwischen der Kortikalis im Bereich der Bohrung und dem Nagelschaft sich ändert. Der Flansch bildet dabei ein Widerlager für die Kortikalis beim Aufbringen der axialen Vorspannung.

Besonders vorteilhaft ist die Anwendung der Erfindung bei Nagelschäften, deren Querbohrung ein Gewinde aufweist, das mit dem Gewinde der Verriegelungsschraube zusammenwirkt. Mit Hilfe einer derartigen Konstruktion wird nicht nur eine axiale und Drehposition vorgegeben, sondern auch die seitliche Lage des Nagels festgelegt.

Bei der Anwendung auf einen Suprakondylamagel ist erfindungsgemäß vorgesehen, dass eine erste Vorspannhülse zwischen dem Kopf der Kondylenschraube und dem Nagelschaft und eine zweite Vorspannhülse zwischen dem Nagelschaft und der Kondylenmutter angeordnet ist. Eine derartige Ausbildung verhindert zum einen das Lockerwerden der Schraubverbindung der Kondylenschraube und zum anderen ein seitliches Ausweichen des Nagelschaftes (Scheibenwischereffekt).

Es sind verschiedene konstruktive Möglichkeiten denkbar, mit Hilfe einer metallischen Hülse eine Federwirkung in axialer Richtung zu erzielen. Eine sieht erfindungsgemäß vor, die Verriegelungshülse mit einer Reihe von axial beabstandeten in Umfangsrichtung versetzten Schlitzen zu versehen. Die Schlitze überlappen sich vorzugsweise in Umfangsrichtung und erstrecken sich vorzugsweise über einen Winkel von mehr als 180°. Der Versatz zwischen benachbarten Umfangsschlitzen kann nach einer weiteren Ausgestaltung der Erfindung zum Beispiel 90° betragen.

Die Erfindung soll nachfolgend anhand von zwei Ausführungsbeispielen näher erläutert werden.
- Fig. 1: zeigt schematisch einen Verriegelungsnagel in einem Humerus zur Versorgung des Humeruskopfes mit einer Vorspannhülse nach der Erfindung.
- Fig. 2: zeigt schematisch den distalen Femur mit zwei Vorspannhülsen und einer Kondylenschraube.
- Fig. 3: zeigt perspektivisch eine Vorspannhülse nach der Erfindung.
- Fig. 4: zeigt einen Schnitt durch die Vorspannhülse nach Fig. 3.

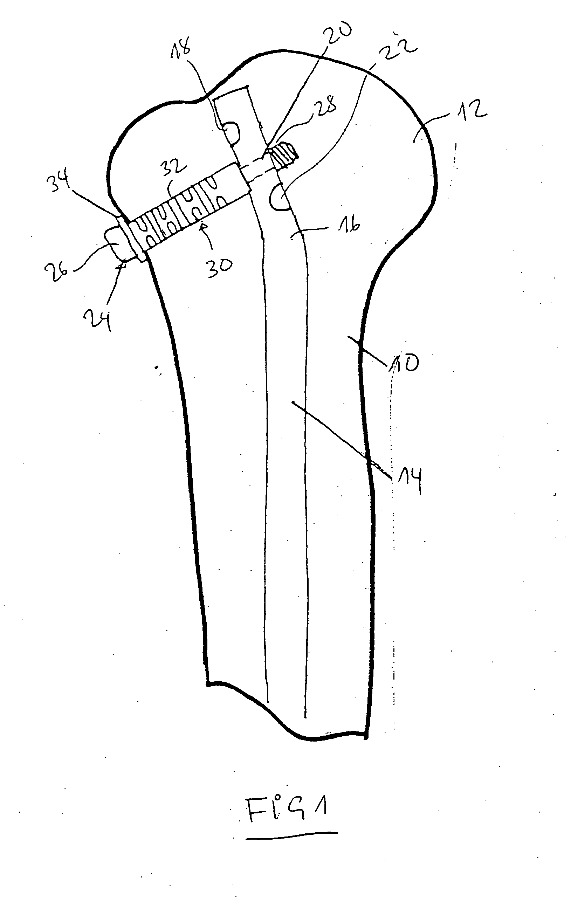

In Fig. 1 ist der proximale Teil eines Humerus angedeutet und mit dem Bezugszeichen 10 versehen. Der Humeruskopf weist das Bezugszeichen 12 auf. Über den Kopf 12 ist ein Verriegelungsnagel 14 von herkömmlichem Aufbau eingesetzt. Er ist bei 16 gekrümmt. Im proximalen Teil weist er drei Querbohrungen 18, 20 und 22 auf, die in einem Winkel zueinander versetzt sind und axial beabstandet liegen. Sie sind mit einem Gewinde (nicht gezeigt) versehen.

Die Querbohrungen 18 bis 22 dienen zur Aufnahme einer Verriegelungsschraube, von denen eine bei 24 dargestellt ist. Sie weist einen Kopf 26 auf und einen Schaft 28, der einen Gewindeabschnitt aufweist. Der Gewindeabschnitt sitzt im Gewinde der Querbohrung 20.

Auf dem Schaft 28 der Verriegelungsschraube 24 ist eine Hülse 30 angeordnet, die einen in axialer Richtung liegenden Schaft 32 aufweist und an einem Ende einen radial umlaufenden Flansch 34.

Bei der Versorgung wird die Kortikalis des Humerus 10 so weit aufgebohrt, dass die Vorspannhülse 30 hindurchgeführt werden kann, wobei der Flansch 34 gegen die Außenseite der Kortikalis zur Anlage kommt. Der Kopf 26 der Verriegelungsschraube 24 kommt in Anlage an den Flansch 34, wenn die Verriegelungsschraube eingeschraubt wird. Dies geschieht in der Weise, dass die Hülse 30 unter axiale Vorspannung gesetzt wird, d.h. in axialer Richtung geringfügig kontrahiert wird. Vergrößert sich der Abstand zwischen dem Nagel 14 und der Kortikalis, sorgt die Vorspannhülse 30 dafür, dass die Verriegelungsschraube noch ausreichend unter Spannung steht und daran gehindert ist, auszuwandern.

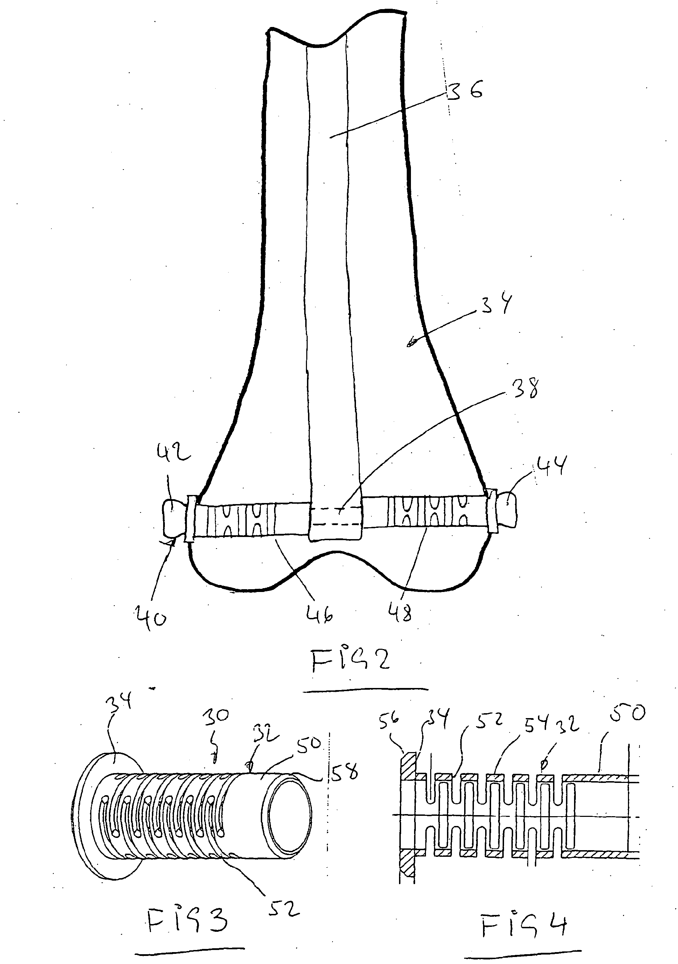

In Fig. 2 ist ein distaler Femurabschnitt bei 34 angedeutet. Von der Unterseite zwischen den Kondylen ist, wie an sich bekannt, ein Verriegelungsnagel 36 eingetrieben. Er weist wie üblich mindestens eine untere und im oberen Bereich mindestens eine obere Querbohrung (nicht gezeigt) auf. Die untere Querbohrung ist mit 38 bezeichnet. Durch diese hindurch führt der nicht weiter zu erkennende Schaft einer Kondylenschraube 40, die einen Kopf 42 aufweist. Auf das Ende der Kondylenschraube 40, die auf der gegenüberliegenden Seite des entsprechenden Kondylus herausragt, ist eine Kondylenmutter 44 aufgeschraubt. Dadurch ist der Schaft des Nagels 36 sowohl in axialer als auch in Drehrichtung festgelegt. Zwischen dem Nagelschaft 36 und dem Kopf 42 der Kondylenschraube 40 einerseits und dem Schaft und der Kondylenmutter 44 andererseits ist jeweils eine Vorspannhülse 46 bzw. 48 angeordnet. Ihr Aufbau gleicht dem der Hülse 30 nach Fig. 1.

Mit Hilfe der beschriebenen Anordnung können die Vorspannhülsen 46, 48 axial vorgespannt werden und sorgen dafür, dass auch bei Lockerung der Verschraubung keine Verlagerung des Schaftes stattfindet oder ein Wandern der Schraubenteile.

In den Figuren 3 und 4 ist ein Ausführungsbeispiel einer derartigen Hülse, z.B. der Hülse 30, dargestellt. An dem dem Flansch 34 gegenüberliegenden Ende ist ein glatter Hülsenabschnitt 50 vorgesehen. Zwischen dem Abschnitt 50 und dem Flansch 34 ist eine Reihe von axial beabstandeten Umfangsschlitzen 52 vorgesehen, die sich über mehr als 180° um den Umfang der Hülse erstrecken und jeweils einander um 90° überlappen. Die Breite der Schlitze ist etwas geringer als die Breite der Stegabschnitte 54 zwischen benachbarten Schlitzen 52. Bei der Auswahl des geeigneten Materials und entsprechenden Dicke lässt sich dadurch eine ausreichende Federwirkung erzielen.

Wie aus Fig. 4 zu erkennen, weist der Ringflansch 34 an der dem Schaft 32 gegenüberliegenden Kante eine umlaufende Fase 56 auf. Der glatte Abschnitt 50 ist am freien Ende ebenfalls mit einer Fase 58 versehen, um das Einführen zu erleichtern.

## Patentansprüche

1. Osteosynthesehilfsmittel für Röhrenknochen, aufweisend:
- eine Verriegelungsschraube mit Kopf;
- einem Verriegelungsnagel;
wobei der Verriegelungsnagel einen Schaft und an beiden Enden des Schaftes mindestens eine Querbohrung für die Verriegelungsschraube aufweist, **dadurch gekennzeichnet, dass** das Osteosynthesehilfsmittel ferner
- eine Vorspannhülse (30) aufweist;
wobei die Vorspannhülse (30) zwischen dem Kopf (26) der Verriegelungsschraube (24) und dem Schaft (14) des Verriegelungsnagels angeordnet ist; und wobei die Vorspannhülse (30) zum Aufbringen einer axialen Spannung eine Reihe von axial beabstandeten Umfangschlitzen (52) aufweist, die in Umfangsrichtung zueinander versetzt sind.

2. Osteosynthesehilfsmittel nach Anspruch 1, **dadurch gekennzeichnet, dass** die Hülse (30) an einem Ende einen radialen Flansch (34) aufweist, gegen den der Kopf (26) der Verriegelungsschraube (24) zur Anlage kommt.

3. Osteosynthesehilfsmittel nach Anspruch 1 oder 2, **dadurch gekennzeichnet, dass** die Verriegelungsschraube einen Gewindeabschnitt (28) aufweist, der mit einem Gewinde einer Querbohrung zusammenwirkt.

4. Osteosynthesehifsmittel nach einem der Ansprüche 1 bis 3, **dadurch gekennzeichnet, dass** eine Kondylenschraube (40) mit Kopf (42) vorgesehen ist, auf deren dem Kopf (42) abgewandtem Ende eine Kondylenmutter (44) schraubbar ist und eine erste Vorspannhülse (46) zwischen dem Kopf (42) der Kondylenschraube (40) und dem Nagelschaft (36) und eine zweite Vorspannhülse (48) zwischen dem Nagelschaft (36) und der Kondylenmutter (44) angeordnet ist.

5. Vorspannhülse (30,46,48), insbesondere für ein Osteosynthesehilfsmittel nach einem der Ansprüche 1 bis 4, **dadurch gekennzeichnet, dass** die Vorspannhülse (30,46,48) zum Aufbringen einer axialen Spannung zwischen dem Kopf einer Verriegelungs- oder Kondylcnschraube und dem Schaft eines Verriegelungsnagels eine Reihe von axial beabstandeten Umfangsschlitzcn (52) aufeist, die in Umfangsrichtung zueinander versetzt sind.

6. Vorspannhülse nach Anspruch 5, **dadurch gekennzeichnet, dass** die Endabschnitte der Umfangsschlitze (52) einander überlappen.

7. Vorspannhülse nach Anspruch 6, **dadurch gekennzeichnet, dass** die Umfangsschlitze (52) sich um einen Winkel von mehr als 180° erstrecken.

8. Vorspannhülse nach Anspruch 7, **dadurch gekennzeichnet, dass** die Umfangsschlitze (52) um etwa 90° zueinander versetzt angeordnet sind.

9. Vorspannhülse nach einem der Ansprüche 5 bis 8, **dadurch gekennzeichnet, dass** sie an einem Ende einen radialen Ringflansch (34) aufweist.

10. Vorspannhülse nach Anspruch 9, **dadurch gekennzeichnet, dass** der Ringflansch (34) auf seiner dem Schaft (32) entgegen gesetzten Seite eine Fase (56) aufweist.

## Claims

1. An osteosynthetic aid for tubular bones, comprising:
a locking screw having a head;
a locking nail;
wherein the locking nail has a shank and, at both ends of said shank, has at least one cross-bore for the locking screw,
**characterized in that** the osteosynthetic aid further comprises
a biasing sleeve (30),
wherein the biasing sleeve (30) is arranged between the head (26) of the locking screw (24) and the shaft (14) of the locking nail (30), and wherein the biasing sleeve (30) comprises a series of axially spaced circumferential slots (52) which are circumferentially offset from each other, for applying an axial tension.

2. The osteosynthetic aid as set forth in claim 1, **characterized in that** the sleeve (30) has a radial flange (34) at one end against which the head (26) of the locking screw (24) comes to bear.

3. The osteosynthetic aid as set forth in claim 1 or 2, **characterized in that** the locking screw has a threaded portion (28) which interengages with a thread of a cross-bore.

4. The osteosynthetic aid as set forth in any one of claims 1 to 3, **characterized in that** a condyle screw (40) with a head (42) is provided, wherein a condyle nut (44) is screwable onto an end of the screw opposite to the head (42), and a first biasing sleeve (46) is disposed between the head (42) of the condyle screw (40) and the nail shank (36) and a second biasing sleeve (48) is disposed between the nail shank (36) and the condyle nut (44).

5. A biasing sleeve (30, 46, 48) for an osteosythetic aid according to any one of claims 1 to 4, **characterized in that** the biasing sleeve (30, 46, 48) comprises a series of axially spaced circumferential slots (52) which are circumferentially offset from each other, for applying an axial tension between a head of a locking screw or condyle screw and a shank of the locking nail.

6. The biasing element as set forth in claim 5, **characterized in that** end portions of the circumferential slots (52) overlap each other.

7. The biasing element as set forth in claim 6, **characterized in that** the circumferential slots (52) extend through an angle of more than 180°.

8. The biasing element as set forth in claim 7, **characterized in that** the circumferential slots (52) are disposed to be offset by about 90° from each other.

9. The biasing element as set forth in any one of claims 5 to 8, **characterized in that** the sleeve has a radial annular flange (34) at one end.

10. The biasing element as set forth in claim 9, **characterized in that** the annular flange (34) has a chamfer (56) on its side opposite the nail shank (32).

## Revendications

1. Auxiliaire d'ostéosynthèse pour des os longs, présentant :
- une vis de verrouillage avec tête ;
- un clou de verrouillage ;
le clou de verrouillage présentant une tige et au moins un trou transversal pour la vis de verrouillage aux deux extrémités de la tige,
**caractérisé en ce que** l'auxiliaire d'ostéosynthèse présente en outre
- une douille de précontrainte (30) ;
la douille de précontrainte (30) étant disposée entre la tête (26) de la vis de verrouillage (24) et la tige (14) du clou de verrouillage ; et la douille de précontrainte (30) présentant, pour l'application d'une tension axiale, une rangée de fentes périphériques (52) distantes axialement, qui sont en déport mutuel dans la direction circonférentielle.

2. Auxiliaire d'ostéosynthèse suivant la revendication 1, **caractérisé en ce que** la douille (30) présente à une extrémité une butée radiale (34), au contact de laquelle arrive la tête (26) de la vis de verrouillage (24).

3. Auxiliaire d'ostéosynthèse suivant l'une des revendications 1 et 2, **caractérisé en que** la vis de verrouillage présente une section filetée (28), qui coopère avec un taraudage d'un trou transversal.

4. Auxiliaire d'ostéosynthèse suivant l'une des revendications 1 à 3, **caractérisé en ce qu'**il est prévu une vis condylienne (40) avec tête (42), vis sur l'extrémité de laquelle, opposée à la tête (42), peut être vissé un écrou condylien (44), et une première douille de précontrainte (46) est disposée entre la tête (42) de la vis condylienne (40) et la tige de clou (36) et une seconde douille de précontrainte (48) est disposée entre la tige de clou (36) et l'écrou condylien (44).

5. Douille de précontrainte (30, 46, 48), en particulier pour un auxiliaire d'ostéosynthèse suivant l'une des revendications 1 à 4, **caractérisée en ce que** la douille de précontrainte (30, 46, 48) présente, pour l'application d'une tension axiale entre la tête d'une vis de verrouillage ou condylienne et la tige d'un clou de verrouillage, une rangée de fentes périphériques (52) distantes axialement, qui sont en déport mutuel dans la direction circonférentielle.

6. Douille de précontrainte suivant la revendication 5, **caractérisée en ce que** les sections extrêmes des fentes périphériques (52) se chevauchent les unes les autres.

7. Douille de précontrainte suivant la revendication 6, **caractérisée en ce que** les fentes périphériques (52) s'étendent sur un angle de plus de 180°.

8. Douille de précontrainte suivant la revendication 7, **caractérisée en ce que** les fentes périphériques (52) sont disposées en déport mutuel d'environ 90°.

9. Douille de précontrainte suivant l'une des revendications 5 à 8, **caractérisée en ce qu'**elle présente à une extrémité une butée annulaire radiale (34).

10. Douille de précontrainte suivant la revendication 9, **caractérisée en ce que** la butée annulaire (34) présente un chanfrein (56) sur son côté opposé à la tige (32).
